# FASCICULE DE BREVET EUROPEEN

(11) **EP 4 203 985 B1**
(45) Date de publication et mention de la délivrance du brevet: **24.09.2025**
(21) Numéro de dépôt: 21777576.6
(22) Date de dépôt: 31.08.2021
(51) Int. Cl.: A61K 36/28, A61K 8/9789, A61P 17/00, A61Q 19/00

(54) **HUILE DE SILYBUM MARIANUM (L.) GAERTN. DANS LE RENFORCEMENT DE LA FONCTION BARRIÈRE DE LA PEAU**
VERWENDUNG VON SILYBUM MARIANUM (L.) GAERTN. ÖL ZUR STÄRKUNG DER BARRIEREFUNKTION DER HAUT
USE OF SILYBUM MARIANUM (L.) GAERTN. OIL IN STRENGTHENING THE BARRIER FUNCTION OF THE SKIN

(30) Priorité: 31.08.2020 FR 2008818
(43) Date de publication de la demande: 05.07.2023
(73) Titulaire: Pierre Fabre Dermo-Cosmétique, 81500 Lavaur (FR)
(72) Inventeur: LETI, Mathieu, 31450 Baziege (FR); JACQUES JAMIN, Carine, 31170 TOURNEFEUILLE (FR)
(74) Mandataire: Regimbeau
(86) Numéro de dépôt international: PCT/FR2021/051499
(87) Numéro de publication internationale: WO 2022/043641

(56) Documents cités:
- EP-A1- 4 009 997
- WO-A1-2018/002338
- WO-A1-2019/122203
- WO-A1-2021/023820
- WO-A2-2012/092597
- ANDRZEJEWSKA J ET AL: "Silybum marianum: non-medical exploitation of the species", ANNALS OF APPLIED BIOLOGY, ASSOCIATION OF APPLIED BIOLOGISTS, WELLESBOURNE, GB, vol. 167, no. 3, 18 June 2015 (2015-06-18), pages 285 - 297, XP071069277, ISSN: 0003-4746, DOI: 10.1111/AAB.12232
- GIORDANO MARIA ET AL: "Silybum marianum: not Just Silymarin and Flavonolignans-", RECORDS OF NATURAL PRODUCTS, vol. 15, no. 4, 17 April 2021 (2021-04-17), TU, pages 243 - 253, XP093216150, ISSN: 1307-6167, DOI: 10.25135/rnp.219.20.09.1827
- ANONYMOUS: "Akène - Wikipédia", 9 February 2024 (2024-02-09), pages 1 - 3, XP093216309, Retrieved from the Internet <URL:https://fr.wikipedia.org/wiki/Akène>
- DATABASE GNPD [online] MINTEL; 23 March 2020 (2020-03-23), ANONYMOUS: "Regeneration Hypoallergenic Face Cream with Toning Birch Juice", XP093216315, retrieved from https://www.gnpd.com/sinatra/recordpage/7457215/ Database accession no. 7457215
- DATABASE GNPD [online] MINTEL; 28 July 2020 (2020-07-28), ANONYMOUS: "Cream Gel", XP055815584, retrieved from https://www.gnpd.com/sinatra/recordpage/7984761/ Database accession no. 7984761
- DATABASE GNPD [online] MINTEL; 8 April 2020 (2020-04-08), ANONYMOUS: "Comedomed Anti-Blemishes Concentrate", XP055815597, retrieved from https://www.gnpd.com/sinatra/recordpage/7540133/ Database accession no. 7540133
- DATABASE GNPD [online] MINTEL; 24 February 2016 (2016-02-24), ANONYMOUS: "Surface Recovery Treatment", XP055815603, retrieved from https://www.gnpd.com/sinatra/recordpage/3833485/ Database accession no. 3833485
- DATABASE GNPD [online] MINTEL; 17 December 2018 (2018-12-17), ANONYMOUS: "Sensitive After Shave Balm", XP055815607, retrieved from https://www.gnpd.com/sinatra/recordpage/6072523/ Database accession no. 6072523

## Description

### DOMAINE TECHNIQUE DE L'INVENTION

La présente invention concerne l'utilisation d'une huile issue d'akènes de *Silybum marianum* (L.) Gaertn. et/ou l'utilisation de compositions cosmétiques comprenant une telle huile, ainsi qu'une méthode cosmétique pour prévenir la diminution et/ou renforcer la fonction barrière épidermique.

### ETAT DE LA TECHNIQUE

Le nom scientifique *Silybum marianum* (L.) Gaertn. désigne une plante appartenant à la famille des Asteraceae, annuelle ou bisannuelle à tige robuste pouvant atteindre plus d'un mètre de hauteur. Ses grandes feuilles luisantes, alternées, sans stipule sont marbrées de blanc et bordées d'épines dures et pointues. Les fleurs sont groupées en capitules terminaux, souvent solitaires. Ils sont entourés de grandes bractées épineuses à l'extrémité très acérée. Les fleurs, tubulées, à cinq lobes, sont de couleur pourpre violacé. Les fruits sont des akènes luisants, noirs ou marbrés de jaune, surmontés d'une aigrette à soies denticulées en anneau à leur base. Le principal nom vernaculaire de cette plante est Chardon-Marie. Cette plante affectionne particulièrement les lieux secs et ensoleillés, souvent sur sols acides, secs et cailleux. Sa répartition géographique est concentrée sur le pourtour méditerranéen, mais elle est également présente en Europe, en Asie de l'Ouest, ainsi qu'en Amérique du Nord et en Australie ou même en Nouvelle-Zélande. Elle pousse dans les jardins mais elle est plus dominante dans les champs incultes, dans les pâturages, le long des bordures des sentiers et entre les décombres.

L'akène (souvent dénommé graine de manière erronée dans la littérature) de *Silybum marianum* (L.) Gaertn. et ses préparations sont traditionnellement utilisés par voie orale, dans le traitement symptomatique des troubles fonctionnels digestifs attribués à une origine hépatique.

Le principe actif principal de l'akène de *Silybum marianum* (L.) Gaertn. est la silymarine, qui est un mélange de plusieurs flavonolignanes. La silymarine contient majoritairement (au moins 95% en poids) un mélange des quatre flavonolignanes suivants : silybine, isosilybine, silychristine et silydianine (Kuki et al., Chromatographia 2012, 75, 175-180). Les akènes contiennent jusqu'à 3% en poids de silymarine. Ils sont également constitués d'huile (15-30% en poids), de mucilages et de protéines.

La silymarine a fait l'objet de nombreuses études (*in vitro, in vivo* et cliniques) ayant démontré ses propriétés antioxydante, hépatoprotectrice, digestive, ou encore anti-inflammatoire. Actuellement, des extraits d'akènes de *Silybum marianum* (L.) Gaertn. titrés en silymarine sont présents dans plusieurs préparations pharmaceutiques destinées au traitement de divers troubles hépatiques et biliaires, telles que le Legalon^{®}.

L'effet antiprolifératif de la silybine a été étudié dans un modèle de cellules HepG2 issues d'un hépatocarcinome. Il a été montré que la silybine induisait une augmentation significative de la synthèse de certains céramides qui peuvent agir comme second messager dans différents processus apoptotiques (Zappavigna et al., Int. J. Mol. Sciences 2019, 20, 2190). Une augmentation de la synthèse des céramides a également été mise en évidence dans le même modèle cellulaire par les silybines A et B et des dérivés synthétiques, la 3-O-galloyl silybine A et la 3-O-galloyl silybine B (Boojar et al., Iranian J. Pharmaceutical Res. 2016, 15(3), 421-433).

Les akènes de *Silybum marianum* (L.) Gaertn. contiennent généralement 15 à 30 % d'huile. L'élimination de l'huile des akènes (déshuilage) est une étape préalable à l'extraction de la silymarine. L'huile de *Silybum marianum* (L.) Gaertn. est à ce titre un co-produit de la production de silymarine (Zhu et al., Biochemidice and Pharmacotherapy 2018, 100, 191-197). L'huile de *Silybum marianum* (L.) Gaertn. est donc dépourvue de silymarine ou en contient des traces non détectables. Cela est confirmé par l'analyse de la fraction polyphénols de l'huile de chardon-Marie qui ne révèle en effet la présence d'aucun constituant de la silymarine (Meddeb et al., Antioxidants, 2018, 7, 95 ; Zarrouk et al., Current Pharmaceutical Design, 2019, 25, 1791-1805).

L'huile non raffinée de *Silybum marianum* (L.) Gaertn. est essentiellement composée de triglycérides d'acides gras insaturés dont les majoritaires sont l'acide linoléique (30 à 60%) et l'acide oléique (15 à 30 %). Sa teneur élevée en acides gras insaturés lui permet d'entrer dans les régimes anti-cholestérol et d'être utilisée dans la prévention des maladies cardio-vasculaires (El-Mallah et al., Grasas y Aceites 2003, 54(4), 397-402). L'huile contient également des acides gras saturés : acide palmitique (5 à 15%), acide stéarique (3 à 8 %), acide arachidique (1 à 4 %) et acide béhénique (1 à 4 %). L'huile brute obtenue par pression à froid contient également des phytostérols (beta-sitosterol notamment) et des tocophérols (α-tocophérol, et γ-tocophérol notamment) (Dabbour et al., Pakistan Journal of Nutrition 2014, 13(2), 67-78).

L'huile de *Silybum marianum* (L.) Gaertn. est essentiellement utilisée dans le domaine culinaire.

Par ailleurs, des études sur les propriétés antioxydante et hépatoprotectrice de l'huile de *Silybum marianum* (L.) Gaertn., administrée par voie orale, ont été réalisées *in vivo* sur des rats ou des souris (Hermenean et al., Open Life Sci. 2015, 10-225-236 ; Zhu et al., Pharmacogn Mag 2014, 10(Sup 1), S92-S99).

Plusieurs études *in tubo* ou *in vitro* mettent en évidence les propriétés antioxydantes et cytoprotective de l'huile de *Silybum marianum* (L.) Gaertn. obtenue par pression à froid ou par extraction par solvant (Dabbour et al., 2014 ; Harrabi et al., Lipids in Health and Disease 2018, 17, 82 ; Meddeb et al., Antioxidants 2018 7, 95).

Une étude clinique met en évidence l'effet anti-âge sur peau âgée et par application topique répétée de 2 formulations cosmétiques contenant 1% d'huile de *Silybum marianum* (L.) Gaertn. Une amélioration des rides faciales, de la densité du derme, de l'élasticité et du teint de la peau est observée après applications 2 fois par jour durant 2 semaines. Toutefois, ces formulations contiennent plusieurs ingrédients actifs comme des palmitoyl peptides, de la vitamine E, de l'huile de jojoba, de l'huile d'avocat, des glycosphingolipides, et du hyaluronate de sodium (Hahn et al., Experimental and Therapeutic Medicine 2016, 12, 1171-1176).

L'utilisation d'huile de *Silybum marianum* (L.) Gaertn. comme biocarburant est également considérée (Takase et al., Ultrasonics Sonochemistry 2014, 21, 1752-1762).

La peau est constituée de différents tissus formant une barrière vitale pour l'organisme vis-à-vis de l'environnement extérieur. Cette barrière protège l'organisme contre les agressions extérieures, notamment chimiques, mécaniques ou infectieuses, et à ce titre un certain nombre de réactions de défense contre les facteurs environnementaux et/ou les xénobiotiques, se produisent à son niveau.

La peau est constituée de trois parties principales, l'une superficielle, l'épiderme, la partie interne, le derme et une couche plus profonde, l'hypoderme, qui interagissent.

L'épiderme humain se compose de quatre à cinq couches distinctes (selon le site anatomique) et de quatre types de cellules qui sont les kératinocytes, très majoritaires, les mélanocytes, les cellules de Langherans et les cellules de Merkel. Chacun de ces types cellulaires contribue par ses fonctions propres au rôle essentiel joué dans l'organisme par la peau, notamment le rôle de protection de l'organisme des agressions extérieures. Cette propriété est appelée fonction barrière.

Les cellules épidermiques prolifèrent au niveau de sa couche la plus profonde, la couche basale, et se différencient au cours de leur migration vers les couches supérieures pour former successivement la couche épineuse constituée de plusieurs couches de cellules polyédriques disposées sur les couches germinatives, la couche granuleuse constituée de cellules aplaties contenant des inclusions cytoplasmiques distinctes, les grains de kératohyaline et enfin la couche cornée (ou *stratum corneum*) qui est la couche la plus superficielle de l'épiderme. La couche cornée est constituée de 20 à 30 couches de kératinocytes au stade terminal de leur différenciation appelés cornéocytes. Les cornéocytes, éléments constitutifs de la couche cornée, sont des cellules mortes, plates, contenant de l'eau et de la kératine. L'architecture du *stratum corneum* est classiquement assimilé à un mur de briques. Les briques représentent les cornéocytes. Les cornéocytes sont entourés d'un « ciment » lipophile constitué de lipides. La fonction barrière est principalement assurée par le *stratum corneum* de par sa structure et sa composition. Au cours de la différenciation des kératinocytes, les phospholipides dont le rôle consiste à élaborer la structure fluide des membranes cellulaires des couches vivantes de l'épiderme, sont peu à peu remplacés par un mélange composé en majeure partie d'acides gras, de cholestérol et de sphingolipides (céramides). Ces lipides, qui sont organisés en bicouches lamellaires, forment le ciment intracellulaire du *stratum corneum.* L'organisation supramoléculaire des lipides intercornéocytaires joue un rôle primordial dans l'établissement des propriétés physico-chimiques du *stratum corneum* et par conséquent dans le maintien d'un gradient hydrique physiologique. La structure de ces bicouches lipidiques possède des propriétés d'assemblage particulières, soit hexagonale (état gélifié), soit orthorhombique (système cristallin dont la maille élémentaire est un parallélépipède rectangle), cette dernière étant majoritaire (Bouwstra et al., Int. J. Cosmet. Sci., 2008, 30, 388). L'état orthorhombique représente la conformation la plus dense et un équilibre entre ces deux états est nécessaire pour des propriétés de barrière optimale. Un dérèglement dans la proportion des trois familles de lipides du *stratum corneum* entraine une modification des états orthorhombique et hexagonal et par conséquent une modification de la fonction barrière. Cette structure en bicouches lamellaires alterne des zones hydrophiles et lipophiles qui conditionnent la fonction barrière, les échanges d'eau entre l'organisme et le milieu extérieur, ainsi que l'hydratation du *stratum corneum.*

Ce dernier a longtemps été considéré comme une simple couche de cellules mortes sans réelle fonction. En réalité, elle est métaboliquement active et assure en très grande partie la fonction de barrière de l'épiderme.

L'épiderme n'est irrigué par aucun vaisseau sanguin et est seulement alimenté par diffusion depuis le derme.

Le derme fournit à l'épiderme un support solide. Le derme est un tissu conjonctif composé de différents types cellulaires dont les fibroblastes, les lymphocytes et les macrophages. On trouve associé à ces cellules des fibres de collagène et l'élastine, incluses dans un gel appelé « substance fondamentale ». Le collagène et l'élastine sont synthétisés par les fibroblastes. On y trouve aussi des leucocytes, des mastocytes ou encore des macrophages tissulaires. Enfin, le derme est traversé par des vaisseaux sanguins et des fibres nerveuses, notamment des fibres sensorielles libres ou reliées à des capteurs.

La cohésion entre l'épiderme et le derme est assurée par la jonction dermo-épidermique. L'équilibre de la barrière cutanée et des muqueuses est dépendant de mécanismes biologiques complexes faisant intervenir de nombreux facteurs de croissance, hormones, enzymes et médiateurs au sein de l'épiderme et du derme.

Enfin l'hypoderme est la couche la plus profonde et la plus épaisse de la peau. Il s'inscrit dans la continuité du derme sans réelle séparation entre les deux tissus. L'hypoderme constitue un coussin amortisseur qui fait office de protection mécanique pour les structures sous-jacentes. Cette couche graisseuse permet également d'isoler le corps des variations thermiques. Si le derme peut être considéré comme une réserve d'eau, les graisses stockées au sein des adipocytes de l'hypoderme constituent une réserve d'énergie.

Il est manifeste que la qualité de la barrière cutanée et des muqueuses est dépendante de mécanismes biologiques endogènes complexes faisant intervenir de nombreux facteurs de croissance, de différenciation, des molécules d'adhésion, des hormones et des enzymes du métabolisme lipidique.

Ainsi, une altération de la barrière cutanée et/ou une rupture de la continuité de la surface de la peau peut se produire en présence d'agressions extérieures de type agents irritants (détergents, acides, bases, oxydants, réducteurs, solvants concentrés, gaz ou fumées toxiques), sollicitations mécaniques (frottements, chocs, abrasion, arrachement de la surface, projection de poussières, de particules, rasage ou épilation), déséquilibres thermiques ou climatiques (froid, sécheresse, radiations), ou xénobiotiques (micro-organismes indésirable, allergènes) ou d'agressions internes de type stress psychologique. Ces agressions provoquent des carences lipidiques, en particulier pour les céramides. Ces modifications de ratio lipidiques vont modifier l'organisation du ciment lipidique et entrainer une altération de la fonction barrière augmentant la perte insensible en eau et une modification des facteurs naturels d'hydratation. Ces modifications vont entrainer une déshydratation de la peau ainsi qu'une peau sèche et pourront également aggraver les cas de dermatite atopique, les sensations de peau sensibles ou réactives.

Cette altération de la barrière cutanée peut notamment se traduire par un inconfort cutané, des phénomènes sensoriels et notamment des phénomènes désagréables. Cette sensation d'inconfort cutané peut se manifester notamment par des picotements, des tiraillements, des échauffements, des démangeaisons. Ces sensations d'inconfort cutané sont plus fréquentes dans les zones les plus exposées de l'organisme, à savoir les mains, les pieds, le visage et le cuir chevelu. Elles peuvent survenir notamment sur des zones soumises à certains gestes d'hygiène quotidienne ou fréquemment renouvelés tels que le rasage, l'épilation, la détersion par des produits de toilette ou des produits ménagers, l'application d'adhésifs avec des pansements ou des patchs, la fixation de prothèses ou dans le cas de gestes sportifs, professionnels ou simplement liés au mode de vie et à l'utilisation de vêtements, d'outils ou d'équipements générant des frottements localisés. Elles peuvent également être amplifiées par le stress psychologique.

L'altération de la barrière cutanée peut aussi favoriser l'apparition de micro-gerçures ou microfissures, en particulier au niveau des mains, des pieds et des lèvres.

Ces sensations d'inconfort cutané concernent toutes les personnes et en particulier celles qui ont des peaux sensibles, voire intolérantes. La notion de peau sensible reflète le niveau de sensibilité de la peau de chacun. S'il est possible d'avoir une peau sensible à tout âge, cela est extrêmement répandu chez les bébés et les personnes âgées. La peau des bébés a une épaisseur représentant environ un cinquième de celle de la peau des adultes. Elle est par conséquent extrêmement sensible aux agressions chimiques, physiques et microbiennes, ainsi qu'aux rayons UV. La fonction de barrière de la peau des adultes, quant à elle, s'affaiblit progressivement avec l'âge, parallèlement au ralentissement des processus métaboliques. Le vieillissement de la peau entraine peu à peu une déficience en lipides, ce qui la rend plus facilement irritable par les substances alcalines telles que le savon.

Lorsque la peau présente un seuil de sensibilité très bas, c'est-à-dire qu'elle réagit de manière exacerbée à la moindre agression extérieure, on parlera de peau intolérante, voire de peau intolérante réactive. Les peaux intolérantes sont plus vulnérables aux agressions extérieures et se caractérisent par un inconfort quotidien et une forte irritabilité. Certains signes, plus ou moins marqués, permettent de les reconnaitre. La peau intolérante du visage présente par exemple des rougeurs et des picotements, elle tire, chauffe ou démange, elle peut également procurer des sensations de brûlure. Les peux intolérantes présentent généralement un terrain allergique et sont par conséquent, particulièrement sensibles aux composants des soins cosmétiques.

La peau sensible est en fait une peau sujette aux picotements, échauffements, fourmillements et démangeaisons, parfois accompagnées de rougeurs. Ces sensations d'inconfort apparaissent de façon exacerbée en réaction à des stimuli qui ne déclencheraient pas d'irritation sur une peau dite normale. Cette hyper-sensibilité de la peau résulte d'une diminution de son seuil de tolérance. Plus la peau est sensible, plus son seuil de tolérance est faible et lorsque le seuil de tolérance est au plus bas, on parlera de peau intolérante. Cette hyper-sensibilité peut s'expliquer par différents facteurs, mais le plus important est une altération de la fonction barrière de l'épiderme. Ce phénomène favorise alors une déshydratation de la peau et surtout la pénétration d'agents potentiellement irritants.

La peau est recouverte d'un film protecteur, appelé film hydrolipidique. Il constitue la barrière la plus externe, ainsi que la plus fragile, et la plus facilement perturbée. Il est constitué en grande partie des corps gras excrétés par les glandes sébacées et des lipides provenant de la dégradation des cellules (squalène, cires, triglycérides, acides gras libres, esters de cholestérol) lors de la kératinisation des cellules cornées, ainsi que de composés hydrophiles, tels que l'eau de la sueur, le glycérol, l'urée, les facteurs naturels d'hydratation de la peau, les sels, les métabolites de la flore cutanée. Ce film de surface est très exposé et très sensible aux stress environnementaux, aux habitudes d'hygiène, et à l'état de la peau ainsi qu'à l'exposition aux rayonnements UV. Le microbiote peut de façon importante modifier la composition du sébum, dégrader les triglycérides et modifier les ratios en acides gras libres, en particulier lors d'un stress ou d'une pathologie. Il s'avère donc important de préserver et même d'améliorer cette fonction barrière cutanée, et ce d'autant plus pour les peaux les plus sensibles.

Il existe toujours un besoin d'agents pour prévenir une diminution de et/ou renforcer la fonction barrière de la peau.

### RESUME DE L'INVENTION

De façon surprenante et inattendue, les inventeurs ont mis en évidence que l'huile issue d'akènes de *Silybum marianum* (L.) Gaertn. induit une synthèse cutanée de lipides, notamment une synthèse endogène de céramides ce qui permet, outre l'effet nourrissant et/ou hydratant pour la peau, de renforcer la fonction barrière épidermique ou de prévenir une diminution de cette fonction barrière épidermique mais également de renforcer la protection de la peau contre la perte en eau et/ou les agressions extérieures.

Selon un premier aspect, l'invention concerne l'utilisation cosmétique non-thérapeutique d'une huile issue d'akènes de *Silybum marianum* (L.) Gaertn. pour augmenter les céramides totaux dans la peau afin de prévenir une diminution et/ou renforcer la fonction barrière épidermique.

Selon un autre aspect, l'invention concerne également une huile issue d'akènes de *Silybum marianum* (L.) Gaertn, pour son utilisation dans la prévention et/ou le traitement des irritations de la peau, par l'augmentation des céramides totaux dans la peau afin de prévenir une diminution et/ou de renforcer la fonction barrière épidermique.

Selon un autre aspect, l'invention concerne une composition cosmétique comprenant au moins une huile issue d'akènes de *Silybum marianum* (L.) Gaertn et au moins un excipient cosmétiquement acceptable, pour son utilisation dans la prévention et/ou le traitement des irritations de la peau, par l'augmentation des céramides totaux dans la peau afin de prévenir une diminution et/ou de renforcer la fonction barrière épidermique

### Définitions

Dans la présente invention, la plante *Silybum marianum* (L.) Gaertn. pourra être désignée de manière abrégée par le terme *Silybum marianum.*

Par « solvant organique non miscible à l'huile issue d'akènes de *Silybum marianum* »*,* on entend, au sens de la présente invention, un solvant organique qui n'est pas capable de se mélanger, ou seulement partiellement, avec l'huile issue d'akènes de *Silybum marianum,* de sorte que le mélange du solvant organique et de l'huile issue d'akènes de *Silybum marianum* donne un mélange hétérogène dans lequel il peut être observé au moins deux phases distinctes.

Par « solvant apolaire », il faut comprendre, au sens de la présente invention, un solvant choisi par exemple parmi l'heptane, l'hexane, le limonène, les hydrocarbures halogénés (par ex. hydrocarbures chlorés en C₁ à C₃ tels que le chloroforme ou le dichlorométhane), le CO₂ supercritique, un mélange CO₂ supercritique et éthanol, et les mélanges de ces solvants. On peut citer également les solvants 100% biosourcés comme par exemple EcoXtract LIPOCOS (Fournisseur Pennakem Europa).

Par « raffinage », on entend au sens de la présente invention les étapes de désodorisation et/ou décoloration et/ou désolvantation de l'huile de *Silybum marianum.* En effet, les huiles brutes renferment un certain nombre de constituants responsables du goût et d'odeurs désagréables et de leur mauvaise conservation, qu'il peut donc être souhaitable d'enlever. Par « désodorisation », on entend au sens de la présente invention un traitement visant à éliminer l'odeur ou le goût d'une huile végétale. La désodorisation peut se faire en chauffant l'huile à haute température (par ex. entre 150 et 300°C, notamment entre 150 et 250°C ou encore entre 150 et 200°C), sous vide, avec injection de vapeur d'eau.

Par « décoloration », on entend au sens de la présente invention un traitement visant à abaisser la coloration de l'huile végétale. La mesure de la couleur peut se faire selon la mesure de Gardner. L'échelle de couleur Gardner est une échelle de comparaison visuelle de la couleur des liquides clairs et transparents. La décoloration peut être obtenue par mise en contact de l'huile avec une terre décolorante (qui permettra d'absorber les pigments (e.g. carotène, chlorophylle...) responsables de la coloration) et un chauffage (par ex. entre 60 et 100°C) qui peut se faire sous vide. Avantageusement, une étape de filtration ultérieure permettra de séparer l'huile et de la terre décolorante désormais usée.

Par « désolvantation », on entend au sens de la présente invention un traitement permettant d'éliminer le solvant présent dans une huile. La désolvantation peut se faire par distillation en chauffant le mélange sous vide et/ou par entrainement à la vapeur d'eau sous vide.

Par « alcool en C₁ à C₃ », on entend, au sens de la présente invention, un alcool R-OH dont la chaîne R est une chaîne hydrocarbonée saturée, linéaire, ou ramifiée, comprenant 1 à 3 atomes de carbone. Il pourra s'agir du méthanol, de l'éthanol, du n-propanol ou de l'isopropanol, en particulier du méthanol, de l'éthanol ou de l'isopropanol. De préférence il s'agira de l'isopropanol.

Par « température ambiante », on entend au sens de la présente invention, une température comprise de 15 à 40°C, de préférence de 20 à 30°C, notamment d'environ 25°C.

Par « environ », on entend dans la présente description que la valeur concernée peut être inférieure ou supérieure de 10%, notamment de 5%, en particulier de 2%, plus particulièrement de 1%, à la valeur indiquée.

Par « application topique », on entend au sens de la présente invention une application sur la peau (dont le cuir chevelu), et les muqueuses.

Par « barrière épidermique », on entend au sens de la présente invention les structures cellulaires de l'épiderme, en particulier la barrière tissulaire formée par les cornéocytes et le ciment lipidique intercellulaire.

Par « fonction barrière épidermique », on entend, au sens de la présente invention la fonction protectrice de l'épiderme, notamment contre les agressions extérieures, et la régulation de la perte insensible en eau et des ions.

Par « cosmétiquement acceptable », on entend au sens de la présente invention ce qui est utile dans la préparation d'une composition cosmétique, qui est généralement sûr, non toxique et ni biologiquement ni autrement non souhaitable et qui est acceptable pour une utilisation cosmétique, notamment par application topique au niveau de la peau.

### DESCRIPTION DETAILLEE DE L'INVENTION

Selon un premier aspect, l'invention concerne l'utilisation cosmétique non-thérapeutique d'une huile issue d'akènes de *Silybum marianum* (L.) Gaertn. pour augmenter les céramides totaux dans la peau afin de prévenir une diminution et/ou renforcer la fonction barrière épidermique.

Selon un mode particulier de réalisation, l'invention concerne l'utilisation cosmétique non-thérapeutique d'une huile issue d'akènes de *Silybum marianum* (L.) Gaertn pour renforcer la protection de la peau contre la perte en eau et/ou les agressions extérieures.

Selon un mode particulier de réalisation, l'invention concerne l'utilisation cosmétique non-thérapeutique d'au moins une huile issue d'akènes de *Silybum marianum* (L.) Gaertn pour nourrir et/ou hydrater la peau, incluant le cuir chevelu, et/ou les muqueuses.

Selon un mode particulier de réalisation, l'invention concerne l'utilisation cosmétique non-thérapeutique d'au moins une huile issue d'akènes de *Silybum marianum* (L.) Gaertn pour améliorer la réparation de la peau, en renforçant ou restaurant la fonction barrière.

Selon un mode particulier de réalisation, l'invention concerne l'utilisation cosmétique non-thérapeutique d'au moins une huile issue d'akènes de *Silybum marianum* (L.) Gaertn pour prévenir et/ou diminuer les picotements, les démangeaisons, les tiraillements, les rougeurs de la peau.

Selon un mode particulier de réalisation, l'invention concerne également une huile issue d'akènes de *Silybum marianum* (L.) Gaertn, pour son utilisation dans la prévention et/ou le traitement des irritations de la peau, par l'augmentation des céramides totaux dans la peau afin de prévenir une diminution et/ou de renforcer la fonction barrière épidermique.

L'huile issue d'akènes de *Silybum marianum* sera utilisée plus particulièrement par voie topique, notamment par application sur la peau.

Dans le cadre de la présente invention, l'huile issue d'akènes de *Silybum marianum* est obtenue à partir du fruit (akène), les akènes pouvant être entier ou en morceaux.

Dans le cadre de la présente invention, l'huile issue d'akènes de *Silybum marianum* peut être obtenue par pressage des akènes ou par extraction des akènes avec un solvant apolaire. Dans un mode de réalisation de la présente invention, l'huile issue d'akènes de *Silybum marianum* pourra être obtenue par pression des akènes de *Silybum marianum,* notamment par pression à froid, c'est-à-dire sans chauffage, à température ambiante, suivie d'une étape de filtration.

Dans un mode de réalisation particulier de l'invention, l'huile issue d'akènes de *Silybum marianum* est obtenue par pressage des akènes de *Silybum marianum,* suivie d'une étape de filtration puis de raffinage.

Dans un mode de réalisation particulier de l'invention, l'huile issue d'akènes de *Silybum marianum* est obtenue par pression des akènes de *Silybum marianum,* suivie d'une étape de filtration, puis d'une étape d'extraction avec un solvant d'extraction polaire à moyennement polaire pour éliminer les composés polaires de l'huile, le solvant d'extraction polaire à moyennement polaire comprenant, notamment constitué par, une solution aqueuse hydrotropique, de l'eau subcritique ou un solvant organique non miscible à l'huile issue d'akènes de *Silybum marianum* éventuellement en mélange avec de l'eau, puis éventuellement d'une étape de désolvantation.

Avantageusement, le solvant d'extraction polaire à moyennement polaire comprend, notamment est constitué par, un solvant organique non miscible à l'huile issue d'akènes de *Silybum marianum* éventuellement en mélange avec de l'eau.

Le solvant organique non miscible à l'huile issue d'akènes de *Silybum marianum* pourra être notamment un alcool en C₁ en C₃.

Le solvant d'extraction polaire à moyennement polaire pourra être notamment un alcool en C₁ en C₃ éventuellement en mélange avec de l'eau.

Le solvant organique non miscible à l'huile issue d'akènes de *Silybum marianum,* en particulier un alcool en C₁ à C₃ tel que le méthanol, l'éthanol ou l'isopropanol, pourra être utilisé en mélange avec de l'eau, notamment dans un rapport volumique solvant organique/eau compris entre 80/20 et 100/0, notamment compris entre 85/15 et 95/5, en particulier d'environ 90/10.

Le solvant d'extraction polaire à moyennement polaire pourra notamment être choisi parmi le méthanol, un mélange méthanol/eau, l'éthanol, un mélange éthanol/eau, l'isopropanol et un mélange isopropanol/eau.

Selon un mode de réalisation préféré, le solvant d'extraction polaire à moyennement polaire sera du méthanol, un mélange éthanol / eau dans un rapport volumique d'environ 90/10 ou un mélange isopropanol / eau dans un rapport volumique d'environ 90/10, de préférence un mélange isopropanol / eau dans un rapport volumique d'environ 90/10.

L'étape d'extraction avec un solvant d'extraction polaire à moyennement polaire de l'huile issue d'akènes de *Silybum marianum* sera effectuée en particulier par mélange de l'huile issue d'akènes de *Silybum marianum* avec le solvant d'extraction polaire à moyennement polaire durant 1 à 12 h et en particulier à une température comprise entre 15 et 25°C, notamment d'environ 20°C. La quantité de solvant d'extraction polaire à moyennement polaire utilisé pour effectuer cette extraction sera avantageusement de 0,5 à 3 g, en particulier de 1 à 3 g pour 1 g d'huile issue d'akènes de *Silybum marianum.*

Une phase d'extraction et une phase lipidique (huile résiduelle dépourvue de ses composés polaires appelée encore huile résiduelle épuisée) seront alors obtenues à la fin de cette extraction. La phase lipidique sera avantageusement séparée de la phase d'extraction et récupérée. Elle pourra ensuite être désolvantée, notamment sous vide, pour éliminer le solvant d'extraction polaire à moyennement polaire résiduel et obtenir une huile issue d'akènes de *Silybum marianum* qui est dépourvue de ses constituants polaires (acides gras libres, phytostérols, tocophérols).

Dans un mode de réalisation particulier de l'invention, l'huile issue d'akènes de *Silybum marianum* est obtenue par pression des akènes de *Silybum marianum,* puis extraction avec un solvant d'extraction polaire à moyennement polaire pour éliminer les composés polaires de l'huile comme détaillé ci-dessus, puis désolvantation comme précédemment décrit, puis désodorisation.

Dans un mode de réalisation particulier de l'invention, l'huile issue d'akènes de *Silybum marianum* est obtenue par pression des akènes de *Silybum marianum,* puis extraction avec un solvant d'extraction polaire à moyennement polaire pour éliminer les composés polaires de l'huile comme détaillé ci-dessus, puis désolvantation comme précédemment décrit, puis décoloration.

Dans un mode de réalisation particulier de l'invention, l'huile issue d'akènes de *Silybum marianum* est obtenue par pression des akènes de *Silybum marianum,* puis extraction avec un solvant d'extraction polaire à moyennement polaire pour éliminer les composés polaires de l'huile comme détaillé ci-dessus, puis désolvantation comme précédemment décrit, puis désodorisation et/ou décoloration.

Selon un deuxième aspect, l'invention concerne l'utilisation non-thérapeutique d'une composition cosmétique comprenant au moins une huile issue d'akènes de *Silybum marianum* avec au moins un excipient cosmétiquement acceptable, pour augmenter les céramides totaux dans la peau afin de prévenir une diminution et/ou renforcer la fonction barrière épidermique.

Selon un mode de réalisation particulier, l'invention concerne l'utilisation non-thérapeutique d'une composition cosmétique comprenant au moins une huile issue d'akènes de *Silybum marianum* avec au moins un excipient cosmétiquement acceptable, pour renforcer la protection de la peau contre la perte en eau et/ou les agressions extérieures.

Selon un mode particulier de réalisation, l'invention concerne l'utilisation non-thérapeutique d'une composition cosmétique comprenant au moins une huile issue d'akènes de *Silybum marianum* avec au moins un excipient cosmétiquement acceptable, pour nourrir et/ou hydrater la peau, incluant le cuir chevelu, et/ou les muqueuses.

Selon un mode particulier de réalisation, l'invention concerne l'utilisation non-thérapeutique d'une composition cosmétique comprenant au moins une huile issue d'akènes de *Silybum marianum* avec au moins un excipient cosmétiquement acceptable, pour améliorer la réparation de la peau, en renforçant ou restaurant la fonction barrière. Selon un mode particulier de réalisation, l'invention concerne l'utilisation non-thérapeutique d'une composition cosmétique comprenant au moins une huile issue d'akènes de *Silybum marianum* avec au moins un excipient cosmétiquement acceptable, pour prévenir et/ou diminuer les picotements, les démangeaisons, les tiraillements, les rougeurs de la peau.

Selon un mode particulier de réalisation, l'invention concerne une composition cosmétique comprenant au moins une huile issue d'akènes de *Silybum marianum* (L.) Gaertn et au moins un excipient cosmétiquement acceptable, pour son utilisation dans la prévention et/ou le traitement des irritations de la peau, par l'augmentation des céramides totaux dans la peau afin de prévenir une diminution et/ou de renforcer la fonction barrière épidermique.

Avantageusement l'huile issue d'akènes de *Silybum marianum* comprise dans la composition cosmétique est préparée tel que décrit précédemment.

Dans un mode de réalisation particulier, la composition cosmétique selon l'invention comprend entre 0,01 et 40% en poids par rapport au poids total de la composition, en particulier entre 0,1 et 20% en poids, en particulier entre 0,1 et 10% en poids, plus particulièrement entre 0,1 et 5% en poids, encore plus particulièrement entre 0,1 et 2% en poids, et de façon encore plus particulière entre 0,5 et 1% en poids d'huile issue d'akènes de *Silybum marianum* par rapport au poids total de la composition.

De préférence l'huile issue d'akènes de *Silybum marianum* est présente dans la composition cosmétique à une teneur d'environ 1% en poids par rapport au poids total de la composition. Selon un mode de réalisation particulier, la composition cosmétique selon l'invention ne comprend pas les ingrédients suivants nommés selon la nomenclature INCI : Enteromorpha compressa extract et/ou Ocimum sanctum leaf extract. Ainsi, selon un mode de réalisation particulier, la composition cosmétique selon l'invention ne comprend pas d'extrait d'algue verte et/ou d'extrait de basilic sacré.

Selon un mode de réalisation particulier, la composition cosmétique selon l'invention ne comprend pas d'extrait de fruit de *Momordica grosvenori* et/ou d'extrait de *Pseudopterogorgia elisabethae.*

Selon un mode de réalisation particulier, la composition cosmétique selon l'invention ne comprend pas de Defensil^{®} qui a pour nom INCI : Octyldodecanol (and) Echium Plantagineum Seed Oil (and) Helianthus Annuus (Sunflower) Seed Oil Unsaponifiables (and) Cardiospermum Halicacabum Flower/Leaf/Vine Extract (and) Tocopherol. Ainsi, selon un mode de réalisation particulier, la composition cosmétique selon l'invention ne comprend pas un mélange d'octyldodécanol, d'huile de graines d'*Echium plantagineum* (encore appelée huile de vipérine à feuilles de plantain), de la fraction insaponifiable de l'huile de tournesol, d'un extrait (en particulier de fleurs/feuilles/tiges) de *Cardiospermum Halicacabum* (appelé liane pois de cœur, cœur des Indes, ou encore Poc-poc), et de tocophérol. Selon un mode de réalisation particulier, la composition cosmétique selon l'invention ne comprend pas d'huile de graines d'*Echium plantagineum,* de la fraction insaponifiable de l'huile de tournesol, et/ou d'un extrait (en particulier de fleurs/feuilles/tiges) de *Cardiospermum Halicacabum.*

Selon un mode de réalisation particulier, la composition cosmétique selon l'invention ne comprend pas d'extrait d'algue verte, d'extrait de basilic sacré, d'extrait de fruit de *Momordica grosvenori,* d'extrait de *Pseudopterogorgia elisabethae,* d'huile de graines d'*Echium plantagineum,* de la fraction insaponifiable de l'huile de tournesol, et/ou d'un extrait (en particulier de fleurs/feuilles/tiges) de *Cardiospermum Halicacabum.*

Selon un autre mode de réalisation selon l'invention, la composition cosmétique selon l'invention comprend de l'huile issue d'akènes de *Silybum marianum* comme seul ingrédient actif utile pour nourrir et/ou hydrater la peau, et plus particulièrement utile pour prévenir une diminution et/ou renforcer la fonction barrière épidermique.

Les compositions cosmétiques selon l'invention sont avantageusement destinées à une application topique, notamment par application sur la peau.

Les compositions cosmétiques selon l'invention pourront ainsi se présenter sous les formes qui sont habituellement connues pour une administration topique, c'est-à-dire notamment les lotions, les laits, les émulsions, les sérums, les baumes, les masques, les crèmes, les dispersions, les gels, les mousses ou les sprays.

Avantageusement il s'agira d'un baume et/ou d'un lait.

L'invention vise ainsi des compositions cosmétiques selon l'un des modes de réalisation de la présente invention, caractérisées en ce qu'elles se présentent sous une forme propre et adaptée à une application topique.

Les compositions cosmétiques selon l'invention, outre l'huile issue d'akènes de *Silybum marianum,* et un milieu physiologiquement acceptable, peuvent également contenir des agents tensioactifs, des agents complexants, des conservateurs, des agents stabilisants, des émulsifiants, des épaississants, des gélifiants, des humectants, des émollients, des oligo-éléments, des huiles essentielles, des parfums, des colorants, des agents matifiants, des filtres chimiques ou minéraux, des agents hydratants, des eaux thermales, etc.

Selon un autre aspect, l'invention concerne une composition cosmétique comprenant au moins une huile issue d'akènes de *Silybum marianum* (L.) Gaertn et au moins un excipient cosmétiquement acceptable, pour son utilisation dans la prévention et/ou le traitement des irritations de la peau, par l'augmentation des céramides totaux dans la peau afin de prévenir une diminution et/ou de renforcer la fonction barrière épidermique.

Avantageusement l'huile issue d'akènes de *Silybum marianum* est préparée tel que décrit précédemment et la composition cosmétique est telle que décrite précédemment.

Les exemples qui suivent illustrent l'invention sans en limiter la portée.

### EXEMPLES

### Exemple 1 : Préparation de l'huile issue d'akènes de Silybum marianum obtenue par pression à froid

Pression à froid d'akènes de *Silybum marianum* puis filtration sur filtre presse pour obtenir une huile brute première pression issue d'akènes de *Silybum marianum* (L.) Gaertn.

### Exemple 2 : Préparation de l'huile issue d'akènes de Silybum marianum dépourvue de ses composés polaires et raffinée

Ce procédé se fait en 8 étapes :
- Pression à froid d'akènes de *Silybum marianum* puis filtration sur filtre presse pour obtenir une huile brute première pression issue d'akènes de *Silybum marianum (L.)* Gaertn.
- Extraction de l'huile brute première pression issue d'akènes de *Silybum marianum* par un mélange isopropanol/eau (90/10 v/v) avec 1 poids du mélange isopropanol / eau pour 1 poids d'huile pendant 10 heures à 20°C
- Récupération de la phase lipidique (huile résiduelle épuisée)
- Désolvantation de l'huile résiduelle épuisée : élimination du solvant par évaporation sous-vide (distillation) puis entrainement à la vapeur dans les conditions suivantes :
   Etape 1 - distillation :
      Température de distillation : 90°C
      Durée de distillation : 2 heures
      Vide : 2 mbar progressif
   Etape 2 - strippage :
      Vapeur : environ 7kg/h
      Température de strippage : 90°C
      Durée du strippage : 1 heure
      Vide : 2-20 mbar
- Décoloration de l'huile résiduelle épuisée et désolvantée dans les conditions suivantes :
   Terre naturelle décolorante : Tonsil^{®} 210 FF
   Température de décoloration : 80°C
   Durée de la décoloration : 45minutes
   Vide : 10-20 mbar
- Filtration sur filtre presse
- Désodorisation dans les conditions suivantes :
   Température de désodorisation : 180°C
   Durée de la désodorisation : 2 heures
   Strippage : vapeur (environ 7kg/h)
   Vide : 2-20mbar
- Filtration sur filtre cartouche.

### Exemple 3 : Effets d'huiles issues d'akènes de Silybum marianum sur la synthèse de lipides totaux et de céramides sur une modèle d'épiderme reconstruit

La fonction principale de l'épiderme est de protéger le corps en formant une barrière protectrice vitale contre les agressions extérieures et contre le risque de déshydratation. Le *stratum corneum,* la couche la plus externe de la peau, est responsable en très grande partie de la fonction barrière. Ce *stratum corneum* se compose de cornéocytes incorporés dans une matrice lipidique, dont l'organisation très spécifique dépend de la composition en lipides. Cette dernière est composée d'acides gras libres, de cholestérol et de céramides. Les lipides forment de multiples couches superposées les unes aux autres. Des expériences in vitro ont démontré que la composition lipidique spécifique de la couche cornée permet à elle seule cet arrangement particulier des lipides en bicouches lamellaires (De Jager et al., J. Lipid res. 2005, 46, 2649-2656). Ces lipides jouent un rôle clé dans la fonction barrière de la peau.

Les céramides constituent une famille lipidique de grande importance biologique car ils permettent la cohésion du *stratum corneum* et, par conséquent, la formation de la barrière cutanée. Sur le plan biochimique, ce sont des sphingolipides résultant de l'amidation de la sphingosine avec un acide gras. Ils peuvent être libres ou liés par liaison covalente aux protéines du *stratum corneum.* A l'heure actuelle, 14 classes de céramides ont été identifiées et sont nommées en fonction de leur structure chimique : les céramides peuvent avoir une base sphingosine (S), dihydrosphingosine (dS), phytosphingosine (P), ou 6-hydroxysphingosine (H) à laquelle est liée un acide gras ω-hydroxy (EO ou O), α-hydroxy (A) ou non-hydroxy (N) avec une chaine alkyle de longueur variable. Les céramides EO ont une structure unique car ils possèdent une très longue chaine ω-hydroxy-acide de plus de 34 atomes de carbone liée à un acide linoléique et vont avoir un rôle prépondérant dans l'organisation des bicouches lamellaires du *stratum corneum* et par conséquent sur la fonction barrière.

La quantification des céramides informe sur l'intégrité ou non de la fonction barrière et fournit une valorisation des produits dermo-cosmétiques.

Une grande diversité de céramides entre dans la composition lipidique de la couche cornée. Ils représentent à eux seuls environ la moitié des lipides intercornéocytaires. Les céramides vont avoir un rôle clé dans l'organisation des bicouches lamellaires et en particulier les céramides estérifiés à ultra-longues chaines tels que les céramides EOS, EOP, EOH (Bouwstra et al., Biochim Biophys Acta 1996, 1300(3), 177-186). L'importance des céramides estérifiés, de par leurs très longues chaines carbonées, a été démontrée sur la distance de répétition lamellaire et dans le rangement des chaines (Kessner et al., Chem Phys Lipids, 2010, 163(1), 42-50). De plus, les têtes polaires portées par les céramides en particulier CER EOS et CER EOP, exercent une influence considérable sur ces propriétés structurales requises pour une matrice lipidique fonctionnelle.

Les céramides non-estérifiés sont majoritaires et sont aussi importants pour la fonction barrière mais également pour l'hydratation et la nutrition de la peau. Des études ont montré que, durant l'hiver, des états de sécheresse cutanée ont été corrélés avec une diminution des taux de céramides totaux et plus particulièrement avec les taux de céramides NP et NH (Ishikawa et al., J. Cosmet Dermatol 2013, 12(1), 3-11). Chez les patients atteints de dermatite atopique, il a également été reporté une diminution significative des taux de céramides totaux et plus particulièrement avec les taux de céramides NP, NS et NH et une corrélation inversée avec la mesure de la perte insensible en eau indiquant une altération de la fonction barrière (Ishikawa et al., J Invest Dermatol, 2010, 130(10), 2511-2514). La diminution de céramides estérifiés à chaine ultra-longue ainsi que le raccourcissement des longueurs de chaines des acides gras libres et des céramides en général conduit à une modification de l'organisation lipidique vers un état moins dense (Kessner et al., Skin Pharmacol Physiol 2008, 21(2), 58-74). Cela crée des espaces dans l'arrangement lipidique entre les cornéocytes conduisant à une réduction dans la fonction barrière cutanée et une perméabilité accrue de la peau. Ainsi, une augmentation de ces lipides spécifiques induit une amélioration de la fonction barrière cutanée.

Le but de cette étude est d'évaluer l'impact de l'huile issues d'akènes de *Silybum marianum* sur la synthèse des lipides cutanés, et en particulier sur la synthèse des céramides constituants majeurs du *stratum corneum* d'un point de vue lipidique et d'évaluer l'effet nutritif pour le traitement et l' amélioration de la fonction barrière. Les céramides, les acides gras libres et le cholestérol du *stratum corneum* sont analysés par chromatographie sur couche mince à haute performance (« High-Performance Thin-Layer Chromatography » ou « HPTLC » en anglais). Cette technique rapide est très utilisée pour séparer des mélanges complexes tels que des lipides (Fuchs et al., J. Chromatography A 2011, 1218(19), 2754-2774), la méthode utilisée a fait l'objet d'une publication scientifique (Jamin et al., Eur J Mass Spectrum, 2019, 25(3), 278-290). L'effet d'huile est évalué sur 3 lots d'épidermes reconstruits avec n=3 (triplicate) par condition expérimentale et par lot. Après traitement par l'huile issue d'akènes de *Silybum marianum,* les lipides du *stratum corneum* sont quantifiés par HPTLC afin d'évaluer l'impact du traitement sur la synthèse des lipides cutanés.

### Méthode

Le modèle utilisé dans cette étude est un modèle d'épiderme reconstruit issu d'exérèses cutanées provenant de chirurgie esthétique selon la méthode décrite par Frankart et al. (Frankart et al., Exp. Dermatol. 2012, 21(11), 871-875).

Les cellules (kératinocytes) sont isolées des exérèses cutanées, puis mises en culture avant d'être ensemencées sur des inserts de cultures en immersion dans du milieu de culture puis les inserts de cultures sont mis à l'interface air/liquide dans un incubateur à 37°C dans une atmosphère humidifiée avec 5% de CO₂, pour former le *stratum corneum.*

Il faut 14 jours pour reformer un épiderme reconstruit d'une surface de 0,6cm². Le milieu de culture est changé toutes les 24 heures.

Trois épidermes reconstruits sont utilisés par condition (contrôle, huile issue d'akènes de *Silybum marianum* obtenue par pression à froid, et contrôle positif).

Contrôle : Tween^{®} 20 à 0,01% dans du tampon phosphate pH 7,4 (PBS) ;
Huile issue d'akènes de *Silybum marianum :*
   - 1^{ère} série d'expérimentation : huile obtenue selon l'exemple 1, testée à 1% dans du Tween^{®} 20 à 0,01% dans du tampon phosphate pH 7,4 (PBS) ;
   - 2^{nde} série d'expérimentation : huile obtenue selon l'exemple 2, testée à 1% dans du Tween^{®} 20 à 0,01% dans du tampon phosphate pH 7,4 (PBS) ;
Contrôle positif : crème Dexeryl^{®}.

Au 9^{ème} jour du protocole, les composés à tester sont appliqués une première fois sur les épidermes reconstruits (2mg pour la crème Dexeryl^{®}, 5µl d'huile issue d'akènes de *Silybum marianum* obtenue par pression à froid à 1% dans Tween^{®} 20/PBS ou 5µl de Tween^{®} 20/PBS pour le contrôle par épithélium). Une incubation de 24 heures est réalisée. Une seconde application (mêmes conditions) est réalisée au 10^{ème} jour, avec une incubation de 48 heures.

Une troisième application (mêmes conditions) est réalisée au 13^{ème} jour, avec 24 heures d'incubation.

Enfin une quatrième application (mêmes conditions) est réalisée au 14^{ème} jour (épithélium reconstruit complètement), avec 24 heures d'incubation.

Au 15^{ème} jour, les épidermes reconstruits sont retirés des inserts de culture, le *stratum corneum* est isolé à l'aide de trypsine du reste de l'épiderme. Le *stratum corneum* est ensuite extrait à l'aide de solvants organiques (mélange de chloroforme et méthanol) afin de recueillir les lipides le constituant. Ces lipides sont ensuite concentrés sous azote liquide avant analyse en HPLC

Les céramides, les acides gras libres et le cholestérol du *stratum corneum* sont analysés par HPTLC. Les conditions analytiques sont détaillées ci-dessous, notamment dans le Tableau 1.
Plaque : Lichrospher^{®} HPTLC Silica gel 60 F254S
Dépôt : 6 mm de large, séché sous flux d'azote
Développement : gradient : voir Tableau 1
Post-dérivatisation : solution aqueuse de sulfate de cuivre (10% CuSO₄, 8%H₃PO₄, 5%MeOH)
Détection : λ scanner : 450 nm.

**[Tableau 1]**

| Etapes | Chloroforme | Acétone | MeOH/Eau/ Acide acétique (97/3/1 v/v/v) | Distance (mm) |
|---|---|---|---|---|
| 1 | 81,5 | 4 | 14,5 | 20 |
| 2 | 81,7 | 4 | 14,3 | 30 |
| 3 | 82 | 4 | 14 | 42 |
| 4 | 83 | 4 | 13 | 46 |
| 5 | 84,5 | 4 | 11,5 | 54 |
| 6 | 85 | 4 | 11 | 57 |
| 7 | 86 | 4 | 10 | 59 |
| 8 | 87 | 4 | 9 | 67 |
| 9 | 88 | 4 | 8 | 75 |
| 10 | 90 | 5 | 5 | 83 |
| 11 | 100 | 0 | 0 | 90 |

### Résultats

Il est connu que la crème Dexeryl^{®} augmente la synthèse des lipides. De ce fait, la crème Dexeryl^{®} a été choisie comme contrôle positif et a été appliquée sur les épithéliums reconstruits à 2mg/épithélium.

Plusieurs classes de lipides sont analysées par HPTLC, les acides gras libres, des dérivés du cholestérol (l'oléate de cholestérol et le sulfate de cholestérol) et les céramides.

### 1^{ère} série d'expérimentation

Dans cette première série d'expérimentation, le contrôle positif (crème Dexeryl^{®}) induit, comme il était attendu, une synthèse de lipides, associée à une augmentation des acides gras libres, des dérivés du cholestérol et des céramides. Ces résultats valident les conditions expérimentales.

L'huile issue d'akènes de *Silybum marianum* selon l'exemple 1 n'a aucun effet sur la synthèse des acides gras libres, induit une légère baisse du cholestérol total (-4,3%) mais cette réduction n'atteint pas le seuil de significativité. En revanche, l'huile issue d'akènes de *Silybum marianum* selon l'exemple 1 augmente de façon significative la synthèse des céramides totaux (+ 36,1%). Ce résultat permet de conclure que cette huile issue d'akènes de *Silybum marianum* démontre un effet nourrissant important pour la peau.

Les céramides sont présents comme lipides dominants dans le *stratum corneum,* et jouent un rôle crucial pour la fonction barrière et par conséquent limiter la déshydratation et la rétention d'eau. Sur la base des propriétés importantes des céramides, l'accent a été mis sur différentes sous-classes de céramides, produites par l'application d'huile de *silybum marianum.* En effet, ces céramides n'étaient pas présents dans la formulation, les céramides retrouvés dans le *stratum corneum* correspondent donc uniquement aux céramides produits par la peau.

Le Tableau 2 ci-dessous montre le pourcentage d'induction des céramides produits après l'application de l'huile issue d'akènes de *Silybum marianum* selon l'exemple 1 par rapport au contrôle.

**[Tableau 2]**

| CER AH | CER AP | CER NH | CER AS et EOH | CER AdS et OH | CER NP | CER EOP | CER NdS et NS | CER EOS |
|---|---|---|---|---|---|---|---|---|
| 15,3% | 24,4% | 25,8% | 39,0% | 45,3% | 44,3% | 63,7% | 46,4% | 32,0% |
| P<0,01 | P<0,05 | P<0,05 | P<0,05 | P<0,01 | P<0,05 | P<0,01 | P<0,001 | P<0,05 |

Ainsi, l'huile issue d'akènes de *Silybum marianum,* comparée aux épidermes reconstruits non traités (contrôle), induit une synthèse statistiquement significative de l'ensemble des céramides dans ce modèle d'épiderme reconstruit. Il est intéressant de noter que l'augmentation de la synthèse du céramide CER EOS dépasse 30%, ce céramide étant en quantité réduite en cas d'eczéma et de dermatite atopique, et jouant un rôle important dans la fonction barrière et en particulier l'organisation des bicouches lamellaires. Par ailleurs, les céramides impliqués dans l'organisation lamellaire des lipides dans le *stratum corneum,* c'est-à-dire les céramides estérifiés à ultra longues chaines, comme les céramides EOS, EOH, et EOP, sont augmentés de manière significative à plus de 30% à minima. Les céramides non estérifiés sont également augmentés, comme les céramides NP et NS, qui sont les céramides majoritaires au niveau du *stratum corneum.* Le céramide NP est le céramide majoritaire et contribue de 8-13% aux céramides totaux (Van Smeden et al., J Lipid Res 2011, 52(6), 1211-1221). Ce céramide joue un rôle important pour la formation des bicouches lamellaires avec les céramides estérifiés à ultra longues chaines, ainsi que le céramide AdS qui est lui aussi induit par l'application topique de l'huile issue d'akènes de *Silybum marianum* (Bouwstra et al., Biochim Biophys Acta 1996, 1300(3), 177-186). Le céramide NP avec le céramide NH sont également impliqués dans la sécheresse cutanée lorsque les quantités de ces céramides diminuent (Ishikawa et al., J Cosmet Dermatol 2013, 12(1), 3-11). L'augmentation du taux de céramides totaux et en particulier des céramides qui sont des acteurs majeurs de la fonction barrière comme les céramides EOS, NP et NS, par l'application d'huile issue d'akènes de *Silybum marianum,* est très favorable pour un effet renforçant de la fonction barrière épidermique.

### 2^{nde} série d'expérimentation

Dans cette seconde série d'expérimentation, le contrôle positif (crème Dexeryl^{®}) induit, comme il était attendu, une synthèse de lipides, en particulier des dérivés du cholestérol et des céramides totaux. Il est à noter que la synthèse de lipides est moindre que celle trouvée lors de la première série d'expérience. Ces résultats valident tout de même les conditions expérimentales.

Dans ces conditions, l'huile issue d'akènes de *Silybum marianum* selon l'exemple 2 augmente la synthèse des céramides totaux de l'ordre de 13,6% par rapport aux épidermes reconstruits non traités. Ces résultats démontrent donc un effet nourrissant de cette huile. Comme dans la 1^{ère} série d'expérimentation, l'étude s'est portée sur différentes classes de céramides induits forcément par l'application des produits par la peau, puisque les céramides sont absents des formulations. Les résultats obtenus par l'application de l'huile issue d'akènes de *Silybum marianum* selon l'exemple 2 sont résumés dans le Tableau 3. Le Tableau 3 ci-dessous montre le pourcentage d'induction des céramides produits par l'application de l'huile issue d'akènes de *Silybum marianum* selon l'exemple 2.

**[Tableau 3]**

| CER AH | CER NH | CER AdS et OH | CER NP | CER EOP | CER NdS et NS | CER EOS |
|---|---|---|---|---|---|---|
| 5,9 | 25,8 | 30,5 | 12,7 | 12,8 | 20,7 | 25,7 |
| NSS | P<0,05 | P<0,01 | P<0,05 | P<0,05 | P<0,05 | P<0,05 |

L'huile issue d'akènes de *Silybum marianum* selon l'exemple 2 induit une production statistiquement significative de presque tous les céramides, notamment les céramides qui sont des acteurs majeurs de la fonction barrière.

L'ensemble de ces résultats démontrent que l'huile issue d'akènes de *Silybum marianum a* un effet nourrissant mais est également capable d'induire une synthèse endogène de céramides et donc d'améliorer la fonction barrière épidermique.

## Revendications

1. Utilisation cosmétique non-thérapeutique d'une huile issue d'akènes de *Silybum marianum* (L.) Gaertn. pour augmenter les céramides totaux dans la peau afin de prévenir une diminution et/ou renforcer la fonction barrière épidermique.

2. Utilisation selon la revendication 1, pour renforcer la protection de la peau contre la perte en eau et/ou les agressions extérieures.

3. Utilisation selon la revendication 1, pour nourrir et/ou hydrater la peau, incluant le cuir chevelu et/ou les muqueuses.

4. Utilisation selon la revendication 1, pour améliorer la réparation de la peau, en renforçant ou restaurant la fonction barrière.

5. Utilisation selon la revendication 1, pour prévenir et/ou diminuer les picotements, les démangeaisons, les tiraillements ou les rougeurs de la peau.

6. Utilisation selon l'une quelconque des revendications 1 à 5, **caractérisée en ce que** l'huile issue d'akènes de *Silybum marianum* (L.) Gaertn. est obtenue par un procédé comprenant une étape de pressage à froid des akènes de *Silybum marianum (L.)* Gaertn., suivie d'une étape de filtration.

7. Utilisation selon la revendication 6, **caractérisée en ce que** l'étape de filtration est suivie d'une étape d'extraction avec un solvant d'extraction polaire à moyennement polaire pour donner une phase d'extraction et une phase lipidique, de séparation de la phase d'extraction et de la phase lipidique, et de récupération de la phase lipidique.

8. Utilisation selon la revendication 7, **caractérisée en ce que** le solvant d'extraction polaire à moyennement polaire est un alcool en C1 à C3.

9. Utilisation selon l'une quelconque des revendications 7 et 8, **caractérisée en ce que** la phase lipidique récupérée est ensuite désolvantée, puis éventuellement désodorisée et/ou décolorée.

10. Huile issue d'akènes de *Silybum marianum* (L.) Gaertn. pour son utilisation dans la prévention et/ou le traitement des irritations de la peau, par l'augmentation des céramides totaux dans la peau afin de prévenir une diminution et/ou renforcer la fonction barrière épidermique.

11. Utilisation non-thérapeutique d'une composition cosmétique comprenant au moins une huile issue d'akènes de *Silybum marianum* (L.) Gaertn. avec au moins un excipient cosmétiquement acceptable, pour augmenter les céramides totaux dans la peau afin de prévenir une diminution et/ou renforcer la fonction barrière épidermique.

12. Utilisation d'une composition cosmétique selon la revendication 11, pour renforcer la protection de la peau contre la perte en eau et/ou les agressions extérieures.

13. Utilisation d'une composition cosmétique selon la revendication 11, pour nourrir et/ou hydrater la peau, incluant le cuir chevelu et/ou les muqueuses.

14. Utilisation d'une composition cosmétique selon la revendication 11, pour améliorer la réparation de la peau en renforçant ou restaurant la fonction barrière.

15. Utilisation d'une composition cosmétique selon la revendication 11, pour prévenir et/ou diminuer les picotements, les démangeaisons, les tiraillements ou les rougeurs de la peau.

16. Utilisation d'une composition cosmétique selon l'une quelconque des revendications 11 à 15, **caractérisée en ce qu'**elle contient de 0,1 à 2% en poids d'huile issue d'akènes de *Silybum marianum* (L.) Gaertn. par rapport au poids total de la composition.

17. Composition cosmétique comprenant au moins une huile issue d'akènes de *Silybum marianum* (L.) Gaertn. et au moins un excipient cosmétiquement acceptable, pour son utilisation dans la prévention et/ou le traitement des irritations de la peau, par l'augmentation des céramides totaux dans la peau afin de prévenir une diminution et/ou renforcer la fonction barrière épidermique.

## Patentansprüche

1. Kosmetische nichttherapeutische Verwendung eines Öls aus den Achänen von *Silybum marianum (L.)* Gaertn. zur Erhöhung der Ceramide insgesamt in der Haut zur Vorbeugung einer Verminderung und/oder zur Stärkung der Barrierefunktion der Epidermis.

2. Verwendung nach Anspruch 1 zur Stärkung des Schutzes der Haut vor Wasserverlust und/oder äußeren Einflüssen.

3. Verwendung nach Anspruch 1 zur Pflege und/oder Befeuchtung der Haut, einschließlich der Kopfhaut und/oder der Schleimhäute.

4. Verwendung nach Anspruch 1 zur Verbesserung der Hautreparatur durch Stärkung oder Wiederherstellung der Barrierefunktion.

5. Verwendung nach Anspruch 1 zur Vorbeugung und/oder Verringerung von Kribbeln, Juckreiz, Spannungsgefühl oder Rötungen der Haut.

6. Verwendung nach einem der Ansprüche 1 bis 5, **dadurch gekennzeichnet, dass** das Öl aus den Achänen von *Silybum marianum* (L.) Gaertn. durch ein Verfahren gewonnen wird, das einen Schritt des Kaltpressens der Achänen von *Silybum marianum* (L.) Gaertn. und einen anschließenden Filtrationsschritt umfasst.

7. Verwendung nach Anspruch 6, **dadurch gekennzeichnet, dass** auf den Filtrationsschritt ein Extraktionsschritt mit einem polaren bis mittelpolaren Extraktionslösungsmittel folgt, um eine Extraktionsphase und eine Lipidphase zu erhalten, die Extraktionsphase und die Lipidphase getrennt werden und die Lipidphase zurückgewonnen wird.

8. Verwendung nach Anspruch 7, **dadurch gekennzeichnet, dass** das polare bis mittelpolare Extraktionslösungsmittel ein C1- bis C3-Alkohol ist.

9. Verwendung nach einem der Ansprüche 7 und 8, **dadurch gekennzeichnet, dass** die zurückgewonnene Lipidphase anschließend entsolvatiert und gegebenenfalls desodoriert und/oder entfärbt wird.

10. Öl aus den Achänen von *Silybum marianum (L.)* Gaertn. für seine Verwendung zur Vorbeugung und/oder Behandlung von Hautreizungen durch Erhöhung der Ceramide insgesamt in der Haut zur Vorbeugung einer Verminderung und/oder Stärkung der Barrierefunktion der Epidermis.

11. Nichttherapeutische Verwendung einer kosmetischen Zusammensetzung, umfassend mindestens ein Öl aus den Achänen von *Silybum marianum* (L.) Gaertn. mit mindestens einem kosmetisch akzeptablen Trägerstoff zur Erhöhung der Ceramide insgesamt in der Haut zur Vorbeugung einer Verminderung und/oder zur Stärkung der Barrierefunktion der Epidermis.

12. Verwendung einer kosmetischen Zusammensetzung nach Anspruch 11 zur Stärkung des Schutzes der Haut vor Wasserverlust und/oder äußeren Einflüssen.

13. Verwendung einer kosmetischen Zusammensetzung nach Anspruch 11 zur Pflege und/oder Befeuchtung der Haut, einschließlich der Kopfhaut und/oder der Schleimhäute.

14. Verwendung einer kosmetischen Zusammensetzung nach Anspruch 11 zur Verbesserung der Hautregeneration durch Stärkung oder Wiederherstellung der Barrierefunktion.

15. Verwendung einer kosmetischen Zusammensetzung nach Anspruch 11 zur Vorbeugung und/oder Verringerung von Kribbeln, Juckreiz, Spannungsgefühlen oder Rötungen der Haut.

16. Verwendung einer kosmetischen Zusammensetzung nach einem der Ansprüche 11 bis 15, **dadurch gekennzeichnet, dass** sie 0,1 bis 2 Gew.-% Öl aus den Achänen von *Silybum marianum* (L.) Gaertn., bezogen auf das Gesamtgewicht der Zusammensetzung, enthält.

17. Kosmetische Zusammensetzung, umfassend mindestens ein Öl aus den Achänen von *Silybum marianum* (L.) Gaertn. und mindestens einen kosmetisch akzeptablen Trägerstoff für ihre Verwendung zur Vorbeugung und/oder Behandlung von Reizungen der Haut durch Erhöhung der Ceramide insgesamt in der Haut zur Vorbeugung einer Verringerung und/oder Stärkung der Barrierefunktion der Epidermis.

## Claims

1. Non-therapeutic cosmetic use of an oil from *Silybum marianum* (L.) Gaertn. achenes to increase total ceramides in the skin in order to prevent a decrease and/or strengthen the epidermal barrier function.

2. Use according to claim 1, to strengthen the skin's protection against water loss and/or external aggressions.

3. Use according to claim 1, to nourish and/or moisturize the skin, including the scalp and/or mucous membranes.

4. Use according to claim 1, to improve skin repair by strengthening or restoring the barrier function.

5. Use according to claim 1, to prevent and/or reduce tingling, itching, tightness or redness of the skin.

6. Use according to any of claims 1 to 5, **characterized in that** the oil from *Silybum marianum* (L.) Gaertn. achenes is obtained by a process comprising a cold pressing step of *Silybum marianum* (L.) Gaertn. achenes, followed by a filtration step.

7. Use according to claim 6, **characterized in that** the filtration step is followed by a step of extraction with a polar to medium-polar extraction solvent to give an extraction phase and a lipid phase, separation of the extraction phase and the lipid phase, and recovery of the lipid phase.

8. Use according to claim 7, **characterized in that** the polar to medium-polar extraction solvent is a C1 to C3 alcohol.

9. Use according to any of claims 7 and 8, **characterized in that** the recovered lipid phase is then desolventized and, if desired, deodorized and/or decolorized.

10. Oil from *Silybum marianum* (L.) Gaertn. achenes for use in the prevention and/or treatment of skin irritation by increasing total ceramides in the skin in order to prevent a decrease and/or strengthen the epidermal barrier function .

11. Non-therapeutic use of a cosmetic composition comprising at least one oil from *Silybum marianum* (L.) Gaertn. achenes with at least one cosmetically acceptable excipient, to increase the total ceramides in the skin in order to prevent a decrease and/or strengthen the epidermal barrier function.

12. Use of a cosmetic composition according to claim 11, to strengthen the skin's protection against water loss and/or external aggressions.

13. Use of a cosmetic composition according to claim 11, for nourishing and/or moisturizing the skin, including the scalp and/or mucous membranes.

14. Use of a cosmetic composition according to claim 11, for improving skin repair by strengthening or restoring the barrier function.

15. Use of a cosmetic composition according to claim 11, for preventing and/or reducing skin tingling, itching, tightness or redness.

16. Use of a cosmetic composition according to any of claims 11 to 15, **characterized in that** it contains from 0.1 to 2% by weight of oil from *Silybum marianum* (L.) Gaertn. achenes, relative to the total weight of the composition.

17. Cosmetic composition comprising at least an oil from *Silybum marianum* (L.) Gaertn. achenes with at least one cosmetically acceptable excipient, for its use in the prevention and/or treatment of skin irritations by increasing the total ceramide content in the skin in order to prevent a decrease in and/or strengthen the epidermal barrier function.
